Europäisches Patentamt

European Patent Office

Office européen des brevets

(19)

(11) Veröffentlichungsnummer: **0 064 665**
**B1**

(12) **EUROPÄISCHE PATENTSCHRIFT**

(45) Veröffentlichungstag der Patentschrift:
**10.04.85**

(21) Anmeldenummer: **82103570.6**

(22) Anmeldetag: **27.04.82**

(51) Int. Cl.⁴: **G 01 N 33/92**

(54) **Reagens zur Präzipitation apo-B-haltiger Lipoproteine, Verfahren zu dessen Herstellung und dessen Verwendung bei der Bestimmung von HDL-Cholesterin.**

(30) Priorität: **02.05.81 DE 3117455**

(43) Veröffentlichungstag der Anmeldung:
**17.11.82 Patentblatt 82/46**

(45) Bekanntmachung des Hinweises auf die Patenterteilung:
**10.04.85 Patentblatt 85/15**

(84) Benannte Vertragsstaaten:
**AT BE CH DE FR GB IT LI LU NL SE**

(56) Entgegenhaltungen:
**DE - A - 2 944 138**
**US - A - 4 215 993**

**CLINICAL CHEMISTRY, Band 25, Nr. 4, April 1979 Easton T.-H. GROVE "Effect of Reagent pH on Determination of High-Density Lipoprotein Cholesterol by Precipitation with Sodium Phosphotungstate-Magnesium" Sieten 560 bis 564**
**CLINICAL CHEMISTRY, Band 25, Nr. 6, 1979 Easton G.M. KOSTNER et al. "Determination of High-Density Lipoproteins: Screening Methods Compared" Seiten 939 bis 942**
**JOURNAL OF LIPID RESEARCH, Band 11, 1970 M. BURSTEIN et al. "Rapid Method for the Isolation of Lipoproteins from Human Serum by Precipitation with Polyanions" Seiten 583 bis 595**

(73) Patentinhaber: **BOEHRINGER MANNHEIM GMBH, Patentabteilung, Abt. E Sandhofer Strasse 112-132 Postfach 31 01 20, D-6800 Mannheim 31 Waldhof (DE)**

(72) Erfinder: **Draeger, Brigitte, Dr. rer. nat., Sprungleitenweg 2, D-8132 Tutzing (DE)**
Erfinder: **Ziegenhorn, Joachim, Dr. rer. nat., Ina-Seidel-Weg 1, D-8130 Starnberg (DE)**

**Beschreibung**

Die Erfindung betrifft ein neues Reagens zur Präzipitation apo-B-haltiger Lipoproteine sowie ein Verfahren zu dessen Herstellung. Das Reagens eignet sich besonders zur Abtrennung Apo-B-haltiger Lipoproteine aus einem Lipoprotein-Gemisch. Das Ausfällen der apo-B-Lipoproteine ist vor allem erforderlich, um im Überstand HDL-Cholesterin bestimmen zu können.

High Density Lipoproteine (HLD) sind in den letzten Jahren in den Blickpunkt des Interesses gerückt. Es konnte gezeigt werden, dass eine inverse Beziehung zwischen HDL-Cholesterin und Herzinfarktrisiko besteht. Ist der Anteil an HDL-Cholesterin im Blut gering, so ist mit einem erhöhten Herzinfarktrisiko zu rechnen. Die Möglichkeit zur Bestimmung von HDL-Cholesterin ist ein wesentlicher Beitrag zur Abschätzung des individuellen Atheroskleroserisikos, da bei Kenntnis des HDL-Cholesterinwertes nicht nur eine Aussage über diese schützende Lipoproteinfraktion gemacht werden kann, sondern auch über die bekannte Näherungsformel:

$$\text{LDL-Cholesterin} = \text{Gesamt-Cholesterin} - 1/5\,\text{Gesamt-triglyceride} - \text{HDL-Cholesterin}$$

auf die Menge des stark atherogenen LDL-Cholesterins geschlossen werden kann.

Übliche Voraussetzung für die Bestimmung des Cholesteringehaltes der HDL-Fraktion ist eine Abtrennung aller anderen Lipoproteinklassen. Hierzu gehören Chylomikronen, Very Low Density Lipoproteine (VLDL) und Low Density Lipoproteine (LDL), die zur Klasse der apo-B-haltigen Lipoproteine zählen.

Zur Abtrennung dieser apo-B-haltigen Lipoproteine werden vor allem die Ultrazentrifugation, die Elektrophorese und Präzipitationsmethoden angewandt. Von diesen drei Methoden sind die Fällungsmethoden, bei denen zumeist ein Gemisch aus einem Polyanion und einem zweiwertigen Kation verwendet wird, am praktikabelsten in der Durchführung. Neben der Fällung mit Heparin-Manganionen oder mit Dextransulfat-Magnesiumionen wird sehr häufig die Fällung mit Phosphorwolframat-Magnesiumionen verwendet. Letztere Methode wird beispielsweise in Journal of Lipid Research *11* (1970) S. 583-595 beschrieben.

Zur Durchführung dieser Methode wird eine Phosphorwolframsäurelösung mit Natronlauge auf einen bestimmten pH-Wert eingestellt, vorzugsweise auf pH 6,1-7,6. Die so erhaltene Phosphorwolframatlösung wird der zu messenden Probe, beispielsweise Serum oder Plasma, zugesetzt und zusätzlich eine Magnesiumchloridlösung zugegeben. Die folgenden Mengenverhältnisse sind üblich:

1,000 ml Probe
0,100 ml Phosphorwolframsäurelösung (40 g/l, pH = 6,1-7,6)
0,025 ml Magnesiumchlorid x 6 $H_2O$-Lösung (2 mol/l).

Die apo-B-haltigen Lipoproteine werden auf diese Weise ausgefällt und durch Zentrifugation abgetrennt. Im Präzipitationsüberstand lassen sich dann HDL-Cholesterin und andere HDL-Parameter, wie beispielsweise Apolipoproteine und Phospholipide, bestimmen.

Die Zugabe von Phosphorwolframat- und Magnesiumchloridlösung zur Probe kann auch in Form eines Gemisches erfolgen [vgl. hierzu Clin. Chem. *25*(1979) S. 939-942 und Lipoproteine und Herzinfarkt, Hrsg. H. Greten, P.D. Lang und G. Schettler, Verlag Gerhard Witzrock, Baden-Baden, Köln, New-York (1979) S., 39-44]. Diese Vorgehensweise erhöht die Praktikabilität der Testdurchführung. Nach dieser Variante werden 1,0 ml Probe mit 0,1 ml des Phosphorwolframat - Magnesiumchloridlösungsgemisches versetzt. Im Gegensatz zu den einzelnen Lösungen ist jedoch das Gemisch aus Phosphorwolframat und Magnesiumchloridlösung nicht über einen längeren Zeitraum stabil, da sich Niederschläge im Fällungsreagens bilden.

Aus US-A-4 215 993 ist ferner bekannt zur selektiven Ausfällung der LDL aus menschlichem Serum ein Gemisch aus Phosphorwolframsäure, einem organischen Puffer mit einem Puffer-Bereich von 4.8 bis 6.8 in einer Konzentration von ungefähr 0.2 bis 1.2 M sowie einem neutralen Polymer, ausgewählt aus der Gruppe Polyvinylpyrrolidon, Polyethylenglycol und Poly-N-vinyl-5-methyl-2-oxazolidinon, einzusetzen.

Ein grosser Nachteil der bisher beschriebenen Fällungsmethoden ist ferner, dass bei Triglyceridreichen Proben nach Zentrifugation die Präzipitate teilweise flotieren. Es sind hohe g-Zahlen und lange Zentrifugationszeiten notwendig, um klare Präzipitationsüberstände zu erhalten.

Ein weiterer Nachteil der bekannten Fällungsversion mit Phosphorwolframat/Magnesiumionen liegt darin, dass die Fällung eine Abhängigkeit vom pH-Wert zeigt. Es wird empfohlen, die Fällung bei einem pH-Wert der Phosphorwolframatlösung von 6,1-7,4 durchzuführen. Die Einstellung eines bestimmten pH-Wertes bereitet jedoch erhebliche Schwierigkeiten, da die Phosphorwolframsäure in den bisher zur Fällung verwendeten Konzentrationsbereichen die Glaselektrode zur pH-Messung beeinflusst.

Nachteilig bei den bisherigen Fällungsmethoden ist ferner, dass sich bei dem zuzusetzenden geringen Reagensvolumen handelsübliche Dilutoren, die ein exaktes Abmessen des Reagensvolumens ermöglichen würden, nicht verwenden lassen. Voraussetzung für den Einsatz von Dilutoren ist generell ein Verhältnis von mindestens 2 Volumenteile Fällungsreagens auf 1 Volumenteil Probe.

Aufgabe der vorliegenden Erfindung war es nun, ein neues Reagens zur Fällung von apo-B-haltigen Lipoproteinen bereitzustellen, das die oben erwähnten Nachteile nicht mehr aufweist und für eine präzise, leicht durchführbare Fällung und Zentrifugation der apo-B-haltigen Lipoproteine geeignet ist, so dass eine problemlose, störungsfreie HDL-Cholesterinbestimmung im Zentrifugationsüberstand möglich ist.

Gelöst wird diese Aufgabe durch ein Reagens, das 0,2 - 3 g/l, vorzugsweise 0,25 - 2 g/l Phosphorwolframsäure und mehr als 2 mmol/l, vorzugsweise 4 - 25 mmol/l Magnesiumionen in einem geeigneten Lösungsmittel enthält. Die Magnesiumionen können dem Reagens in Form eines geeigneten Magnesium-

salzes, vorzugsweise als Magnesiumchlorid x 6 H₂O, zugegeben werden. Als Lösungsmittel ist besonders Wasser geeignet.

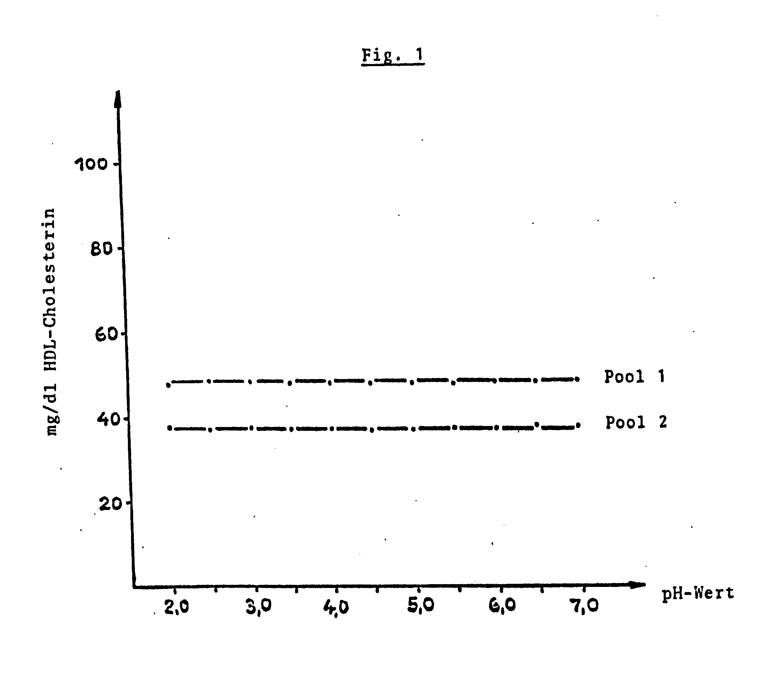

Der wesentliche Vorteil des erfindungsemässen Reagenses liegt darin, dass die nach der Ausfällung apo-B-haltiger Lipoproteine gefundenen HDL-Cholesterinwerte keine pH-Abhängigkeit zeigen (s. Fig. 1). Es ist folglich nicht mehr erforderlich, das Fällungsreagens auf einen bestimmten, dem Stand der Technik entsprechend vorzugsweise neutralen pH-Wert einzustellen. Besonders vorteilhaft wird daher das erfindungsgemässe Fällungsreagens mit einem pH-Wert verwendet, wie er sich beim Auflösen der Phosphorwolframsäure und dem Magnesiumsalz im verwendeten Lösungsmittel ergibt. Dieser pH-Wert liegt beispielsweise bei Verwendung von Magnesiumchlorid x 6 H₂O oder Magnsiumsulfat bei pH 2,2-2,7, bei Verwendung von Magnesiumacetat bei pH 5,4-5,7.

Wie Fig. 1 zeigt, lässt sich mit einem erfindungsemässen Fällungsreagens auch dann noch eine vollständige Präzipitation apo-B-haltiger Lipoproteine erreichen, wenn der pH-Wert des Fällungsreagensgemisches auf höhere Werte eingestellt wird. Dies kann durch Zusatz der erforderlichen Menge einer geeigneten Base, beispielsweise Natronlauge, erfolgen. Limitierend sind jedoch pH-Werte ab etwa 8,0 im Fällungsreagensgemisch, da bei solchen pH-Werten die Magnesiumionen in Form von Magnesiumhydroxid ausfallen. Der pH-Wert des Fällungsreagenses liegt somit vorzugsweise bei 2-8.

Überraschenderweise hat sich gezeigt, dass im Gegensatz zu den bekannten Fällungsreagensgemischen aus Phosphorwolframat und Magnesiumsalzen das erfindungsgemässe Fällungsreagens eine ausgezeichnete Stabilität aufweist. Nach 12 Monaten Lagerung bei 50°C ist das Reagens noch voll funktionsfähig. Niederschlagsbildung im Reagens tritt nicht auf.

Weiterhin wurde überraschenderweise festgestellt, dass mit dem erfindungsgemässen Fällungsreagensgemisch der Lipoprotein-haltige Niederschlag problemlos zentrifugierbar ist. Zur vollständigen Abtrennung des Niederschlags genügt im allgemeinen eine Zentrifugationsdauer von 5 - 10 Min. bei 1.500 g. Dieser Vorteil zeigt sich vor allem bei der Zentrifugation der Präzipitate von Proben mit hohem Lipidgehalt.

In Tabelle 1 sind die HDL-Cholesterin-Werte, gemessen nach Fällung apo-B-haltiger Lipoproteine, einmal mit dem bekannten Fällungsreagens (Probe : Reagens 1,0 : 0,1) und zum andern mit dem erfindungsgemässen Fällungsreagens (Probe : Reagens 1,0 : 2,0) für solche lipämischen Serum-Proben gegenübergestellt.

Tabelle 1

HDL-Cholesterin-Werte lipämischer Proben (hoher Triglycerid-Anteil), gemessen nach Fällen und anschliessender Zentrifugation apo-B-haltiger Lipoproteine

A mit bekanntem Fällungsreagens (Probe : Reagens = 1,0 : 0,1)

(Phosphorwolframat : 40 g/l, mit Natronlauge auf pH 6,1-7,6 eingestellt; Magnesiumchlorid x 6 H₂O : 0,500 mol/l)

B mit erfindungsgemässem Fällungsreagens (Probe : Reagens = 1,0 : 2,0) (1,84 g/l Phosphorwolframsäure; 0,025 mol/l Magnesiumchlorid x 6 H₂O)

| | Gehalt an | | HDL-Cholesterin Fällungsversion | |
| | Gesamt-cholesterin | Tri-glyceride | A | B |
| Serum | [mg/dl] | [mg/dl] | [mg/dl] | [mg/dl] |
|---|---|---|---|---|
| 1 | 170 | 460 | 19,5 | 19,3 |
| 2 | 181 | 467 | 27,5 | 26,8 |
| 3 | 215 | 440 | 41,3 | 41,1 |
| 4 | 197 | 710 | flotiert, trüb | 25,7 |
| 5 | 494 | 522 | 17,7 | 18,1 |
| 6 | 270 | 928 | trüb | 18,1 |
| 7 | 298 | 464 | 21,0 | 23,2 |
| 8 | 257 | 696 | flotiert, trüb | 28,7 |
| 9 | 323 | 1348 | floriert | flotiert |
| 10 | 291 | 1078 | flotiert | 37,4 |
| 11 | 695 | 1566 | flotiert | trüb |
| 12 | 250 | 522 | flotiert, trüb | 30,8 |
| 13 | 273 | 1174 | flotiert | 36,2 |
| 14 | 310 | 652 | flotiert | 30,2 |
| 15 | 261 | 1073 | flotiert | 12,4 |
| 16 | 178 | 681 | flotiert | 20,5 |
| 17 | 221 | 1044 | flotiert, trüb | trüb |
| 18 | 348 | 942 | flotiert | leicht trüb |
| 19 | 216 | 957 | flotiert, trüb | trüb |
| 20 | 262 | 701 | flotiert, trüb | 16,5 |
| 21 | 308 | 464 | flotiert, trüb | 32,7 |
| 22 | | 796 | trüb | 26,5 |
| 23 | | 833 | flotiert | 28,5 |

Die in Tabelle 1 zusammengestellten Werte zeigen, dass Schwierigkeiten beim Fällen apo-B-haltiger Lipoproteine bzw. beim Abzentrifugieren der Präzipitate mit dem erfindungsgemässen Fällungsreagens erst bei wesentlich höherem Lipid-(Triglycerid)-Gehalt auftreten als mit dem bekannten Fällungsreagens. Dies führt zu einem weiteren Vorteil gegenüber der bisher bekannten Phosphorwolframat/Magnesiumsalz-Fällung: eine Verdünnung der lipämischen Proben vor Zugabe des Fällungsreagenses ist erst bei sehr hohen Triglyceridwerten notwendig (oberhalb 1000 mg/dl). Bei den bisher beschriebenen Fällungsvarianten war bereits bei Triglyceridwerten von ca. 400 mg/dl eine Vorverdünnung der zu bestimmenden Probe erforderlich.

Gegenstand der vorliegenden Erfindung ist ferner ein Verfahren zur Herstellung des erfindungsgemässen Reagenses. Dieses Verfahren ist dadurch gekennzeichnet, dass die Phosphorwolframsäure zusammen mit dem Magnesiumsalz in einem geeigneten Lösungsmittel, vorzugsweise Wasser, aufgelöst wird. Man erhält eine Lösung, deren pH-Wert im wesentlichen von dem verwendeten Magnesiumsalz abhängig ist. Die so erhaltene Lösung kann bereits erfindungsgemäss verwendet werden. Gegebenenfalls lässt sich ein anderer pH-Wert durch Zugabe von Natronlauge einstellen. In vorteilhafter Weise werden die Phosphorwolframsäure und das Magnesiumsulfat getrennt in dem Lösungsmittel aufgelöst und die erhaltenen Lösungen anschliessend miteinander vermischt.

Gegenstand der vorliegenden Erfindung ist ferner ein Verfahren zur Präzipitation apo-B-haltiger Lipoproteine aus Körperflüssigkeiten, das dadurch gekennzeichnet ist, dass der zu bestimmenden Probe das erfindungsgemässe Fällungsreagens zugesetzt wird.

Da das erfindungsgemässe Fällungsreagens die Phosphorwolframsäure und die Magnesiumionen in wesentlich geringeren Konzentrationen als die bekannten Fällungsreagentien enthält, kann ersteres in einem wesentlich grösseren Volumen der Probe beigegeben werden, als dies bei den bisher bekannten Fällungsversionen mit Phosphorwolframat/Magnesiumionen der Fall ist. Dies bringt den Vorteil mit sich, dass bei der erfindungsgemässen Präzipitationsmethode ohne weiteres Dilutoren eingesetzt werden können. Der Einsatz von Dilutoren bringt bekanntlich erhebliche Vorteile mit sich, vor allem im Hinblick auf die Praktikabilität der Testdurchführung, beispielsweise grosse Zeitersparnis, verbesserte Präzision, geringerer Materialverbrauch usw. Es findet keine manuelle Dosierung von Probe und Fällungsreagens statt, wodurch die Fehlermöglichkeiten entscheidend verringert werden. Die Volumina lassen sich exakter bestimmen. Die Präzision der Fällung der apo-B-haltigen Lipoproteine und damit der nachfolgenden HDL-Cholesterinbestimmung wird dadurch entscheidend verbessert. Je grösser der Volumenanteil des Fällungsreagenses am Gesamtvolumen des Fällungsansatzes ist, desto geringer sind Verschleppungsprobleme von einem Ansatz zum anderen bei Automatisierung der HDL-Cholesterinbestimmung durch Verwendung von Dilutoren.

Limitierend für den Volumenanteil des Fällungsreagenses am Gesamtvolumen ist die Empfindlichkeit der Methode, die zur anschliessenden Bestimmung der zu messenden HDL-Parameter im Zentrifugationsüberstand zur Verfügung steht. Das für einen bestimmten Einzelfall am besten geeignete Probe : Reagens-Verhältnis kann von einem Fachmann leicht anhand weniger Vorversuche ermittelt werden. Ein Probe : Reagens-Verhältnis von 1 : 2-1 : 20 hat sich für die bisher bekannten Methoden zur Bestimmung der HDL-Parameter als vorteilhaft erwiesen.

Auch die optimalen Konzentrationen der Bestandteile des Fällungsreagenses lassen sich leicht in wenigen Vorversuchen ermitteln. Es hat sich gezeigt, dass bei einem Probe : Reagens-Verhältnis von 1 : 2 ein Fällungsreagens besonders geeignet ist, das 1,8-1,9, vorzugsweise 1,84 g/l Phosphorwolframsäure und 25 mmol/l Magnesiumionen enthält. Wird 1 Volumenteil Probe 2,5 Volumenteile Reagens zugesetzt, so sind 1,4-1,5 g/l, vorzugsweise 1,47 g/l Phosphorwolframsäure und 20 mmol/l Magnesiumionen besonders bevorzugt. Beträgt das Verhältnis Probe : Reagens 1 : 20, so wird vorteilhaft ein Fällungsreagens mit 0,25-0,35 g/l, vorzugsweise

0,306 g/l Phosphorwolframsäure und 4,16 mmol/l Magnesiumionen eingesetzt.

Es ist bekannt, dass manche der Serum- oder Plasmaproben in Folge ihres hohen Triglyceridgehaltes keine klaren Präzipitationsüberstände ergeben oder deren Präzipitate nach Zentrifugation flotieren. Um diese Nachteile zu beseitigen, müssen solche Proben vorverdünnt werden. Es ist bekannt, dass solche vorverdünnten Proben nach Ausfällung der apo-B-haltigen Lipoproteine zu niedrige Werte für HDL-Cholesterin liefern. Es erfolgt eine teilweise Mitfällung von HDL bei der Ausfällung von apo-B-haltigen Lipoproteinen. Bei Verwendung des erfindungsgemässen Fällungsreagenses ist, wie oben bereits erwähnt, eine Vorverdünnung der Probe erst bei höheren Triglyceridgehalten erforderlich als bei Verwendung der bisher bekannten Fällungsmethoden. Die auch bei diesen vorverdünnten Proben festgestellte Mitfällung von HDL lässt sich verhindern, indem dem Fällungsreagens oder dem Verdünnungsmittel der Probe (Wasser oder physiologische Kochsalzlösung) ein geeignetes Detergens zugegeben wird. Als Detergentien in dem erfindungsgemässen Sinne eignen sich vor allem nichtionische und anionische Detergentien. Als nichtionische Detergentien sind bevorzugt Polyoxyäthylenlauryläther, Alkylaryläther, polyoxyäthyliertes Octylphenol, Hydroxypolyäthoxydodecane. Als anionische Detergentien können eingesetzt werden Detergentien vom Sulfosuccinamat oder Sulfosuccimat-Typ wie beispielsweise Tetranatrium-N-(1.2-dicarboxyäthyl)-N-octadecylsulfosuccinamat, Dinatrium-N-octadecylsulfosuccinamat, Natrium-dioctylsulfosuccinat und Dinatriumdodecylsulfosuccinat.

Wird das Detergens dem Fällungsreagens zugesetzt, so haben sich Konzentrationen von 0,03-0,1 % bei nichtionischen Detergentien und 0,1-0,3 % bei den anionischen Detergentien als besonders geeignet erwiesen. Setzt man das Detergens dem Verdünnungsmittel zu, so betragen die Konzentrationen des Detergens im Verdünnungsmittel bei nichtionischen Detergentien 0,1-0,5 % und bei anionischen Detergentien 0,3-1 %.

Die vorliegende Erfindung wird durch die folgenden Beispiele näher erläutert:

*Beispiel 1*

1. Herstellung des Fällungsreagenses

1,84 g Phosphorwolframsäure werden ad 500 ml mit dest. Wasser gelöst; 5,08 g Magnesiumchlorid x 6 H$_2$O werden ad 500 ml mit dest. Wasser gelöst. Beide Lösungen werden miteinander gemischt. Das Reagens ist gebrauchsfertig.

2. Präzipitation apo-B-haltiger Lipoproteine

0,5 ml Probe werden mit 1 ml des Fällungsreagenses vermischt. Nach 5-10 minütiger Zentrifugation bei 1.500 g haben sich alle apo-B-haltigen Lipoproteine als Niederschlag am Boden des Zentrifugationsgefässes abgesetzt. Der klare Präzipitationsüberstand enthält HDL. Im Präzipitationsüberstand lässt sich z.B. HDL-Cholesterin mit einem enzymatischen Cholesterintest bestimmen.

## 3. HDL-Cholesterin-Bestimmung

0,2 ml des Präzipitationsüberstandes werden mit 2 ml einer Reagenslösung zur Cholesterin-Bestimmung versetzt, die aus folgenden Komponenten besteht:

0,2 molarer Phosphatpuffer, pH = 7,8; 1 mmol/l 4-Aminophenazon, 5 mmol/l Phenol, 5 mmol/l 3,4-Dichlorphenol, 0,48% Fettalkoholpolyglykoläther, 0,1 U/ml Cholesterinesterase, 0,14 U/ml Cholesterinoxidase, 0,12 U/ml Peroxidase. Nach 20 Min. Inkubation bei Raumtemperatur wird die Extinktion der Probe bei 546 nm in üblicher Weise gegen den Reagentien-Leerwert gemessen.

Die Berechnung erfolgt über Faktor: mg/dl HDL-Cholesterin = 302,2 · $^{\Delta E}$ Probe.

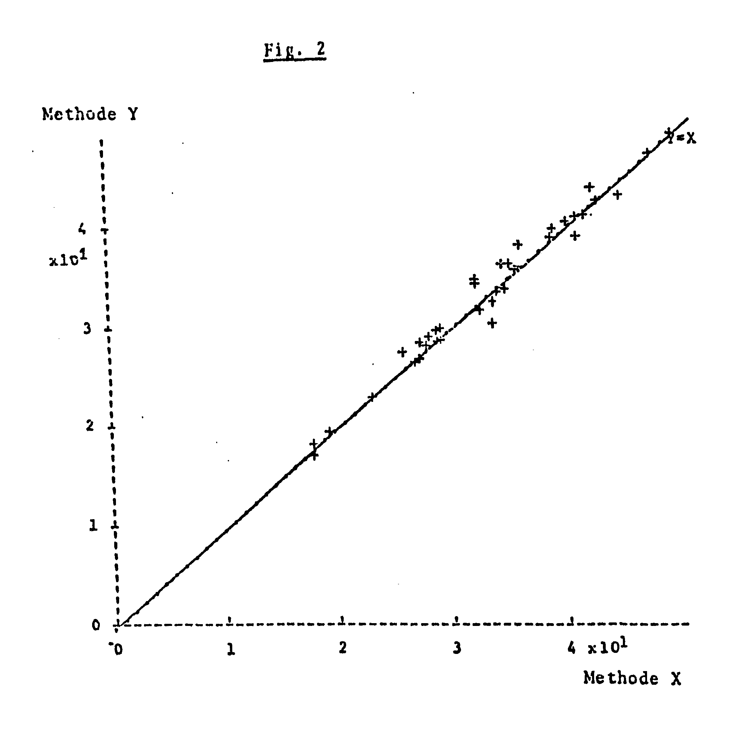

In Fig. 2 sind die Werte, die nach der hier beschriebenen Methode mit einem Verhältnis Probe : erfindungsgemässes Fällungsreagens von 1,0 : 2,0 erhalten worden sind ($\bar{Y}$ = 33,4 mg/dl), gegen die entsprechenden Werte aufgetragen, die sich nach der auf Seite 2 erwähnten Fällungsversion mit einem Verhältnis Probe : bekanntes Fällungsreagens von 1,0 : 0,1 ergeben ($\bar{X}$ = 33,5 mg/dl). Der Fig. 2 kann entnommen werden, dass die nach den beiden Methoden gefundenen Werte gut übereinstimmen.

### Beispiel 2

*Präzipitation apo-B-haltiger Lipoproteine bei einem Probe/Fällungsreagensverhältnis von 1/2,5*

Das in Beispiel 1 unter 1. beschriebene Fällungsreagens wird mit Wasser im Verhältnis 4+1 verdünnt. Das so hergestellte Reagens enthält 1,47 g/l Phosphorwolframsäure und 20 mmol/l Magnesiumchlorid x 6 H₂O.

0,2 ml Probe (Serum, Plasma) werden mit 0,5 ml des verdünnten Fällungsreagenses vermischt. Nach 5-10 minütiger Zentrifugation bei 1500 g erhält man einen klaren Präzipitationsüberstand, der entsprechend Beispiel 3 zur Cholesterin-Bestimmung in den Cholesterin-Test eingesetzt werden kann.

Auch die in der hier beschriebenen Weise erhaltenen HDL-Cholesterin-Werte stimmen gut mit den nach der bekannten Methode gefundenen Werte überein.

### Beispiel 3

*Präzipitation apo-B-haltiger Lipoproteine bei einem Probe/Fällungsreagensverhältnis von 1/20 und anschliessender HDL-Cholesterin-Bestimmung mit einem Cholesterin-Test höherer Empfindlichkeit*

Das in Beispiel 1 unter 1. beschriebene Fällungsreagens wird mit Wasser im Verhältnis 1+5 verdünnt. Man erhält ein Reagens mit 0,306 g/l Phosphorwolframsäure und 4,16 mmol/l Magnesiumchlorid x 6 H₂O.

0,05 ML Probe (Serum, Plasma) werden mit 1 ml des verdünnten Fällungsreagenses vermischt und 5-10 Min. bei 1500 g zentrifugiert.

0,2 ml des klaren Präzipitationsüberstandes werden mit 2 ml der folgenden Reagentien-Lösung vermischt: 0,4 mol/l Phosphatpuffer, pH = 7,7; 2,5 mmol 2,4-Dichlorphenolsulfonsäure; 1 mmol/l 4-Aminophenazon; 1,85 mol/l Methanol; 0,2% Hydroxypolyäthoxydodecan; 0,2 U/ml Cholesterinesterase; 0,1 U/ml Cholesterinoxidase; 0,1 U/ml Peroxidase. Nach 15 Min. Inkubation bei Raumtemperatur wird die Extinktion der Probe bei 546 nm gegen den Reagentien-Leerwert gemessen. Die Auswertung erfolgt über einen 50 mg/dl Cholesterin-Standard, der wie eine Probe behandelt wurde.

In Tabelle 2 sind die auf die hier beschriebene Weise für verschiedene Serum-Proben gefundenen Werte für HDL-Cholesterin den Werten, die nach Fällung mit einem Probe : Reagens-Verhältnis von 1 : 2 (s. Beispiel 1) erhalten worden sind, gegenübergestellt.

### Tabelle 2

HDL-Cholesterin-Werte für verschiedene Serum-Proben nach Fällung apo-B-haltiger Lipoproteine mit unterschiedlichem Verhältnis von Probe : erfindungsgemässem Fällungsreagens

| Serum | Fällung HDL-Cholesterin mit Probe : Reagens-Verhältnis von | |
|---|---|---|
| | 1 : 2 [mg/dl] | 1 : 20 [mg/dl] |
| 1 | 35,2 | 35,2 |
| 2 | 53,4 | 52,8 |
| 3 | 33,9 | 33,2 |
| 4 | 65,8 | 66,9 |
| 5 | 30,0 | 29,3 |

### Beispiel 4

*Präzipitation apo-B-haltiger Lipoproteine mit detergenshaltigem Fällungsreagens*

100 ml des in Beispiel 1 unter 1. beschriebenen Fällungsreagenses werden mit 0,5 ml einer 30%igen wässrigen Lösung von Tetranatrium-N-(1.2 dicarboxyäthyl)-N-octadecylsulfosuccinamat versetzt.

0,5 ml einer 1 : 1 mit Wasser verdünnten Probe werden mit dem detergenshaltigen Fällungsreagens vermischt. Nach Zentrifugation (5-10 Min. bei 1500 g) erhält man einen klaren Präzipitationsüberstand, in dem HDL-Cholesterin bestimmt werden kann.

In Tabelle 3 sind die HDL-Cholesterin-Werte zusammengestellt, die für verdünnte und unverdünnte Proben einmal mit und einmal ohne Detergenszusatz zum Fällungsmittel erhalten worden sind.

### Tabelle 3

HDL-Cholesterin-Werte für eine verdünnte und unverdünnte Probe mit und ohne Detergenszusatz zum Fällungsmittel

| | HDL-Cholesterin | |
|---|---|---|
| | Probe unverdünnt [mg/dl] | Probe 1 : 1 verdünnt [mg/dl] |
| Fällung mit detergensfreiem Fällungsmittel | 46,1 | 42,5 |
| Fällung mit detergenshaltigem Fällungsmittel (Beispiel) | 46,3 | 46,0 |

Die in Tabelle 3 aufgeführten Messwerte zeigen, dass ohne Detergenszusatz in verdünnten Proben zu niedrige HDL-Cholesterin-Werte gefunden werden und dass durch Zusatz eines Detergens dieser Fehler beseitigt werden kann.

Ähnliche Ergebnisse erhält man auch, wenn man in den obigen Beispielen anstelle von Tetranatrium-N--(1.2-dicarboxyäthyl)-N-octadecylsulfosuccinamat auch andere nichtionische oder anionische Detergentien, beispielsweise

Hydroxypolyäthoxydodecan (Thesit)
Polyoxyäthylenlauryläther (Brij 35)
Polyoxyäthylenalkylaryläther (triton-X-100),

dem Fällungsreagens zusetzt (vgl. Tabelle 4). In diesem Falle wurde wegen der geringen Löslichkeit dieser Detergentien im niedrigen pH-Bereich des Fällungsreagenses dessen pH-Wert auf pH = 5,0 erhöht, vorzugsweise durch Zusatz von Natronlauge.

Tabelle 4

HDL-Cholesterin-Werte für verdünnte und unverdünnte Serum-Proben ohne und mit Zusatz verschiedener Detergentien

| | HDL-Cholesterin Fällungsreagens | | | | |
| | ohne | | mit | | |
| Serum | Detergenszusatz | | Detergenszusatz | | |
| | | | Brj 35 0,06% | Thesit 0,04% | Triton-X-100 0,07% |
| | Probe unverdünnt | 1 : 1 verdünnt | Probe 1:1 verdünnt | | |
| | [mg/dl] | [mg/dl] | [mg/dl] | [mg/dl] | [mg/dl] |
| 1 | 45,1 | 42,5 | 45,8 | 47,5 | 46,9 |
| 2 | 45,8 | 40,8 | 46,4 | 46,4 | 46,2 |
| 3 | 34,4 | 31,8 | 33,5 | 34,1 | 34,5 |

*Beispiel 5*

*Präzipitation apo-B-haltiger Lipoproteine nach Verdünnen der Probe mit detergenshaltigem Verdünnungsmittel*

100 ml Wasser werden mit 1,25 ml einer 30%igen Lösung von Tetranatrium-N-(1.2-dicarboxyäthyl)-N--octadecylsulfosuccinamat versetzt.

0,5 ml Probe (Serum, Plasma) werden mit 0,5 ml des detergenshaltigen Wassers vermischt. Zu 0,5 ml dieser Mischung wird 1 ml des in Beispiel 1 unter 1. beschriebenen detergensfreien Fällungsreagenses gegeben. Nach 5-10 minütiger Zentrifugation bei 1.500 g erhält man einen klaren Präzipitationsüberstand.

In Tabelle 5 sind die für unverdünnte, nur mit Wasser verdünnte und mit detergenshaltigem Wasser verdünnte Serum-Proben gefundenen HDL-Cholesterin-Werte zusammengestellt.

Tabelle 5

HDL-Cholesterin-Werte für unverdünnte, nur mit Wasser verdünnte und mit detergenshaltigem Wasser verdünnte Serum-Proben

| | HDL-Cholesterin | | |
| Serum | unver-dünnt | 1 : 1 verdünnt mit Wasser | 1 : 1 verdünnt mit detergens-haltigem Wasser |
| | [mg/dl] | [mg/dl] | [mg/dl] |
| 1 | 40,5 | 30,7 | 41,9 |
| 2 | 53,6 | 43,0 | 55,3 |

Die gefundenen Messdaten zeigen, dass die zu niedrigen HDL-Cholesterin-Werte bei vorverdünnter Probe auch dann vermieden werden können, wenn bereits dem Verdünnungsmittel ein nichtionisches oder anionisches Detergens zugesetzt wird.

Die beigefügten Figuren zeigen im Einzelnen:

Fig. 1 Abhängigkeit der HDL-Cholesterin-Werte von dem pH-Wert nach Fällen der apo-B-haltigen Lipoproteine mit 1,84 g/l Phosphorwolframsäure und 25 mmol/l Magnesiumchlorid x 6 $H_2O$ (Probe : Reagens = 1,0 : 2,0)

Fig. 2 Vergleich der HDL-Cholesterin-Werte, die nach Fällung apo-B-haltiger Lipoproteine mit einem Probe : erfindungsgemässen Fällungsreagens-Verhältnis von 1,0 : 2,0 (Fällungsversion 1,0 : 2,0; Methode Y) und mit einem Probe : bekanntes Fällungsreagens-Verhältnis von 1,0 : 0,1 (Fällungsversion 1,0 : 0,1; Methode X) erhalten worden sind.

**Patentansprüche**

1. Reagens zur Präzipitation apo-B-haltiger Lipoproteine, dadurch gekennzeichnet, dass es in wässriger Lösung 0,2 bis 3 g/l Phosphorwolframsäure und mehr als 2 mmol/l Magnesiumionen enthält und einen pH-Wert von etwa 8,0 oder darunter aufweist.

2. Reagens gemäss Anspruch 1, dadurch gekennzeichnet, dass es 0,25-2 g/l Phosphorwolframsäure und 4-25 mmol/l Magnesiumionen enthält.

3. Reagens gemäss einem der Ansprüche 1 und 2, dadurch gekennzeichnet, dass es einen pH-Wert von 2-8 aufweist, der gegebenenfalls durch Zusatz einer geeigneten Base eingestellt werden kann.

4. Reagens gemäss einem der Ansprüche 1-3, dadurch gekennzeichnet, dass es zusätzlich ein nichtionisches oder anionisches Detergens enthält.

5. Verfahren zur Herstellung des Reagens gemäss einem der Ansprüche 1 bis 4, dadurch gekennzeichnet, dass man die Phosphorwolframsäure und ein geeignetes Magnesiumsalz in Wasser auflöst, gegebenenfalls den gewünschten pH-Wert durch Zusatz einer Base einstellt und gegebenenfalls ein nichtionisches oder anionisches Detergens zusetzt.

6. Verfahren gemäss Anspruch 5, dadurch gekennzeichnet, dass die Phosphorwolframsäure und das Magnesiumsalz getrennt in Wasser gelöst und die beiden Lösungen anschliessend miteinander vermischt werden.

7. Verfahren zur Präzipitation apo-B-haltiger Lipoproteine, dadurch gekennzeichnet, dass man der zu messenden Probe gegebenenfalls nach Zusatz eines Verdünnungsmittels, ein Fällungsreagens gemäss einem der Ansprüche 1-4 zusetzt.

8. Verfahren gemäss Anspruch 7, dadurch gekennzeichnet, dass man 1 Volumenteil Probe mit 2-20 Volumenteile Fällungsreagens versetzt.

9. Verfahren gemäss einem der Ansprüche 7 + 8, dadurch gekennzeichnet, dass dem gegebenenfalls zuzusetzenden Verdünnungsmittel ein nichtionisches oder anionisches Detergens beigemischt ist.

10. Verfahren zur Bestimmung von HDL-Cholesterin, wobei zunächst die apo-B-haltigen Lipoproteine aus der zu messenden Probe ausgefällt werden, der entstehende Niederschlag abzentrifugiert und im Überstand HDL-Cholesterin in an sich bekannter Weise bestimmt wird, dadurch gekennzeichnet, dass man zur Ausfällung der apo-B-haltigen Lipoproteine der zu messenden Probe ein Fällungsreagens gemäss einem der Ansprüche 1-4 zusetzt.

## Revendications

1. Réactif de précipitation pour pré-B-lipoprotéines, caractérisé en ce qu'il contient en solution aqueuse de 0,2 à 3 g/l d'acide phosphotungstique et plus de 2 mmoles/l d'ions de magnésium et en ce qu'il présente un pH d'environ 8,0 ou d'une valeur inférieure.

2. Réactif selon la revendication 1, caractérisé en ce qu'il contient entre 0,25 et 2 g/l d'acide phosphotungstique et entre 4 et 25 mmoles/l d'ions de magnésium.

3. Réactif selon l'une des revendications 1 et 2, caractérisé en ce qu'il présente un pH entre 2 et 8, qui peut éventuellement être obtenu par l'addition d'une base appropriée.

4. Réactif selon l'une des revendications 1 à 3, caractérisé en ce qu'il contient en outre un détergent non ionique ou anionique.

5. Procédé de préparation du réactif selon l'une des revendications 1 à 4, caractérisé en ce qu'on dissout l'acide phosphotungstique et un sel de magnésium approprié dans l'eau, on porte éventuellement au pH souhaité par l'addition d'une base et on ajoute éventuellement un détergent non ionique ou anionique.

6. Procédé selon la revendication 5, caractérisé en ce que l'acide phosphotungstique et le sel de magnésium sont dissous séparément dans l'eau et les deux solutions sont ensuite mélangées ensemble.

7. Procédé de précipitation de pré-B-lipoprotéine, caractérisé en ce qu'on ajoute à l'échantillon à doser éventuellement après l'addition d'un diluant, un réactif de précipitation selon l'une des revendications 1 à 4.

8. Procédé selon la revendication 7, caractérisé en ce qu'on ajoute à une partie en volume d'échantillon entre 2 et 20 parties en volume de réactif de précipitation.

9. Procédé selon l'une des revendications 7 et 8, caractérisé en ce que l'agent diluant à ajouter est additionné d'un détergent non ionique ou anionique.

10. Procédé de dosage de cholestérol HDL dans lequel on élimine d'abord par précipitation les pré-B-lipoprotéines de l'échantillon à doser, on sépare le précipité formé par centrifugation et on dose le cholestérol HDL de façon en soi connue dans le liquide surnageant, caractérisé en ce que pour effectuer la précipitation des pré-B-lipoprotéines, on ajoute à l'échantillon à doser un reactif de précipitation selon l'une des revendications 1 à 4.

## Claims

1. Reagent for the precipitation of apo-B-containing lipoproteins, characterised in that it contains in aqueous solution 0.2 to 3 g./l. of phosphotungstic acid and more than 2 mmole/l. magnesium ions and has a pH value of about 8.0 or below.

2. Reagent according to claim 1, characterised in that it contains 0.25 - 2 g./l. phosphotungstic acid and 4 - 25 mmole/l. magnesium ions.

3. Reagent according to one of claims 1 and 2, characterised in that it has a pH value of 2 - 8, which can if necessary be adjusted by the addition of a suitable base.

4. Reagent according to one of claims 1 - 3, characterised in that it additionally contains a non-ionic or anionic detergent.

5. Process for the preparation of the reagent according to one of claims 1 to 4, characterised in that one dissolves the phosphotungstic acid and a suitable magnesium salt in water, if necessary adjusts the desired pH value by the addition of a base and if necessary adds thereto a non-ionic or anionic detergent.

6. Process according to claim 5, characterised in that the phosphotungstic acid and the magnesium salt are separately dissolved in water and the two solutions are subsequently mixed with one another.

7. Process for the precipitation of apo-B-containing lipoproteins, characterised in that to the sample to be measured, if necessary after the addition of a dilution agent, one adds a precipitation reagent according to one of claims 1 - 4.

8. Process according to claim 7, characterised in that one mixes 1 part by volume of sample with 2 - 20 parts by volume of precipitation reagent.

9. Process according to one of claims 7 and 8, characterised in that a non-ionic or anionic detergent is admixed with the dilution agent possibly to be added.

10. Process for the determination of HDL cholesterol, whereby the apo-B-lipoproteins are first precipitated out of the sample to be measured, the resulting precipitate is centrifuged off and ADL cholesterol is determined in per se known manner in the supernatant, characterised in that, for the precipitating out of the apo-B-containing lipoproteins, one adds to the sample to be measured a precipitation reagent according to one of claims 1 - 4.

## Fig. 1

## Fig. 2

Methode Y

Methode X